# EUROPEAN PATENT SPECIFICATION

(11) **EP 1 937 173 B1**
(45) Date of publication and mention of the grant of the patent: **29.06.2016**
(21) Application number: 06776681.6
(22) Date of filing: 08.08.2006
(51) Int. Cl.: A61B 18/12

(54) **SAFETY DEVICE FOR A HF-SURGERY APPLIANCE**
SICHERHEITSVORRICHTUNG FÜR EIN HF-OPERATIONSGERÄT
DISPOSITIF DE SECURITE POUR UN APPAREIL DE CHIRURGIE A HAUTE FREQUENCE

(30) Priority: 26.08.2005 DE 102005040487
(43) Date of publication of application: 02.07.2008
(73) Proprietor: ERBE Elektromedizin GmbH, 72072 Tübingen (DE)
(72) Inventor: BELLER, Jürgen, 72810 Gomaringen (DE)
(74) Representative: Bohnenberger, Johannes
(86) International application number: PCT/EP2006/007844
(87) International publication number: WO 2007/022864

(56) References cited:
- WO-A-2004/045436
- US-A- 4 171 700
- US-A- 5 152 762
- US-A- 5 496 312

## Description

The invention relates to a safety device for a HF-surgery appliance according to the precharacterizing clause of Claim 1.

In electrosurgical appliances that operate at high frequency - in the following termed HF-surgery appliances - alternating currents in the range of 300 kHz are employed. In this frequency range capacitive and also inductive couplings play a not inconsiderable role.

When several instruments are attached to a HF-surgery appliance, for example one for cutting and another for coagulation, the said capacitive couplings can allow currents to flow within the "inactive" leads. Then, if these leads make contact with the patient or the surgeon, injuries can result.

The IEC standard 60601-1-6 describes measurement procedures with which to determine the limiting value of such a coupled current with respect to the earth potential or to the opposite electrode. This limiting value is 150 mA.

The document DE 35 23 871 C3 discloses a safety circuit for a HF-surgery instrument that is intended to provide means by which, if leakage currents should appear, they can be prevented as far as possible from putting the patient at risk of injury. This known device is relatively elaborate.

WO 2004/045436 A describes a HF-surgery appliance comprising two pairs of connectors to connect two difference instruments to the HF-generator. The HF-surgery appliance is designed such that each of the connector pairs can be connected with a monopolar or bipolar instrument. The document does not describe any safety device which would protect a patient or surgeon from injuries caused by a capacitive coupling of "inactive" leads.

US-A-5496312 shows an automatic controller for electrosurgical HF-generators wherein the impedance between electrodes is measured. The measured impedance value is used to determine the power of the device. A safety device for protecting a patient or a surgeon is not disclosed therein.

The objective of the present invention is to disclose a safety device of the kind cited at the outset that, by simple means, allows leakage currents to be avoided as completely as possible.

This objective is achieved by a safety device according to Claim 1.

In particular, the present safety device for a HF-surgery appliance comprises at least one HF generator with an active output and a neutral output to generate a working current, and at least two working connectors to each of which can be connected, by way of electrical leads, at least one instrument through which the working current can be conducted to a biological tissue, such that the safety device at least reduces the danger presented by unintended "fault currents" that can flow through an inactive instrument, the objective of the invention being achieved by constructing the safety device and connecting it to the working connectors as well as the neutral output in such a way that every working connector through which no working current is flowing is connected to the neutral output.

Hence the crucial.point of the invention resides in the fact that fault currents or leakage currents are not restricted but rather are "annihilated". Thus the risk that injury will be caused by the appliances is reduced.

The safety device preferably comprises switching elements with which to switch a working connector from the neutral output to an active output of the HF generator and to conduct the working current from the HF generator to an instrument. Such change-over switches are preferably constructed as relays and hence are simple to manufacture and to operate.

A control means is provided which preferably is constructed as a microcomputer designed so that in response to an activation signal, the working connector of an instrument to be activated is first connected to an active output of the HF generator, and then the HF generator is triggered so that it delivers the working current. Hence at the outset none of the working connectors are connected to the HF generator; instead, all of them are at the neutral potential. Furthermore, when switching is initiated the working current need not immediately flow through the switch, which instead changes state while in a no-current condition. It is only after switching has occurred that the HF generator is triggered so as to deliver a "signal" set by the user, i.e. the actual working current.

At the working connectors there are provided sampling elements to monitor the current and/or voltage, which are designed to generate a fault-current signal when the current and/or the voltage at a working connector through which no working current is flowing exceeds a pre-adjustable threshold. In this way it is easy to determine whether any fault currents are present. If that is the case, then appropriate measures can be taken. In particular, in such a case (threshold exceeded) a warning signal can be emitted by a signal element to announce an (excessive) fault current. This makes it possible for the user easily to check the operational state of the appliance. When this threshold is reached, or in certain cases a second, higher threshold, the HF generator is switched off by an appropriate control signal, as a result of which the operational safety of the appliance is further increased.

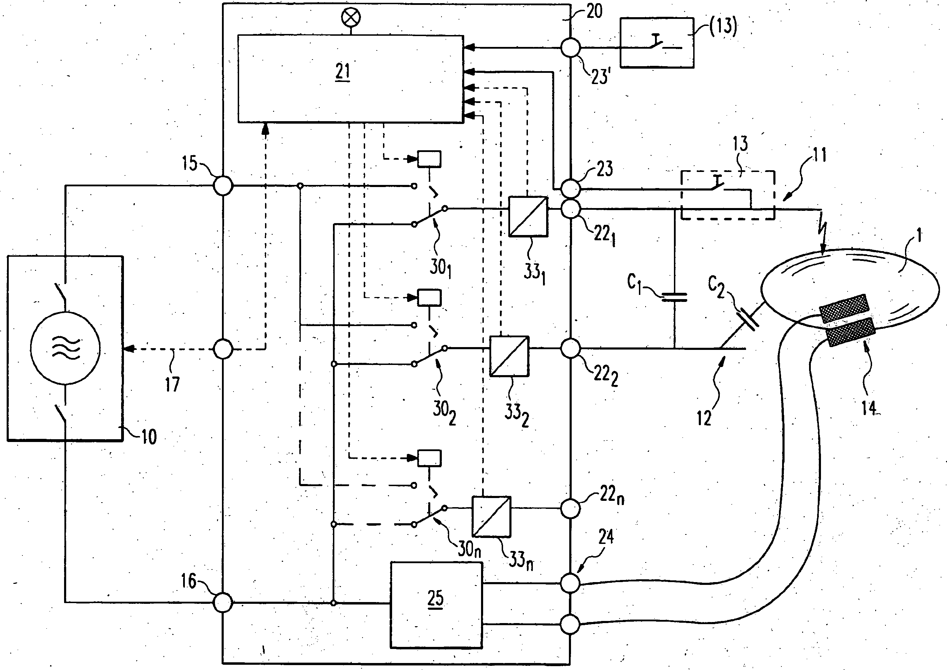

In the following, an exemplary embodiment of the invention is explained in greater detail with reference to the enclosed drawing.

As is evident in the drawing, a high-frequency.generator 10 is provided, which can be adjusted by way of the customary adjustment organs in a manner known per se. The HF generator 10 comprises an active output 15 and a neutral output 16, the latter being connected to an indifferent electrode 14 by way of a test-circuitry component 25 and a connector 24. The indifferent electrode 14 is attached to the patient 1 in such a way as to ensure that only a slight density of current will be transferred from the indifferent electrode 14 into the tissue 1. The safety of the contact is tested by the test circuitry 25.

The signal from the active output 15 is sent to one pole of each of the change-over switches 30₁ to 30ₙ. The second pole of each switch 30, to 30ₙ is connected to the neutral output 16.

The third pole (input) of the change-over switches 30₁ to 30ₙ is connected to working connectors 22₁ to 22ₙ of the safety device 20 by way of transformers 33₁ to 33ₙ. The transformers 33₁ to 33ₙ ascertain which currents are flowing through the working connectors 22₁ to 22ₙ, or which voltages are present at them.

In the exemplary embodiment shown here an active instrument 11, i.e. one that is being used at present, is attached to a first working connector 22₁. In order to allow a highfrequency current to flow, e.g. for the purpose of cutting, the user actuates a switch 13 on the active instrument 11 or a pedal switch (13), each of which is connected by way of a control connector 23 or 23', respectively, to a control means 21 that is preferably designed as a microprocessor. The control means 21 receives the signals from the switch 13 and the transformers 33₁ to 33ₙ. In addition, by way of a control lead 17 the control means 21 is connected to the HF generator 10 in such a way that on one hand the HF generator 10 is controlled by the control means 21, while on the other hand the control means 21 is informed about operational states of the HF generator 10. Furthermore, the control means 21 is in controlling communication with the change-over switches 30, to 30ₙ, which in the present case are constructed as relays, so that in response to relevant signals from the control means 21 the working connectors 22₁ to 22ₙ can be switched by the change-over switches 30₁ to 30ₙ either to the active output 15 or to the neutral output 16.

Thus during employment of the appliance when the switch 13 is actuated, the control means 21 changes the state of the change-over switch 30₁ in such a way that the working connector 22, is applied to the active output 15 of the HF generator 10. The other change-over switches 30₂ to 30ₙ remain in the position shown in the drawing, so that the working connectors 22₂ to 22ₙ remain in communication with the neutral output 16. After a prespecified time has elapsed, a time that is as short as possible while still being long enough to ensure reliable switching of the change-over switches 30, to 30ₙ, the control means 21 controls the HF generator 10 accordingly, so that the latter supplies a working current to-the active instrument 11 by way of the change-over switch 30₁, the transformer 33₁ and the working connector 22₁. The current circuit is closed by the tissue 1, the indifferent electrode 14 and the test circuitry 25, terminating at the neutral output 16 of the HF generator 10.

When the current flows in this way, currents can be coupled into an inactive instrument 12, or its connector fork, by way of capacitive couplings C₁ and C₂, shown schematically in the drawing. When such a current is coupled into the inactive instrument 12, it flows through the working connector 22₂, the transformer 33₂ and the change-over switch 30₂ to the neutral output 16 of the HF generator 10 or to the indifferent electrode 14. This arrangement prevents any damaging current from flowing through the inactive instrument 12, even if it comes into contact with the user or the patient.

If the fault current (measured by the transformer 33₂) exceeds a threshold that has been preset in the control means 21, the latter emits an alarm signal (shown here as a warning lamp). Alternatively or in addition (if a still higher threshold is exceeded) the control means 21 switches off the HF generator 10.

### List of reference numerals

- 1: Tissue/patient
- 10: HF generator
- 11: Active instrument
- 12: Inactive instrument
- 13: Switch
- 14: indifferent electrode
- 15: Active output
- 16: Neutral output
- 17: Control lead
- 20: Safety device
- 21: Control means
- 22₁ to 22ₙ: Working connector
- 23, 23': Control connector
- 24: Connector for indifferent electrode
- 25: Test circuitry
- 30, to 30ₙ: Change-over switch/relay
- 33, to 33ₙ: Transformer

## Claims

1. A HF-surgery appliance comprising:
- at least one HF generator (10) with an active output (15) and a neutral output (16) to generate a working current,
- at least two working connectors (22₁ to 22ₙ) for connection of at least one instrument (11, 12) through which the working current can be conducted to a biological tissue (1),
- a safety device (20) for reducing the danger presented by fault currents that can flow through an inactive instrument (12), the safety device (20) comprising a control means (21) designed such that in response to an activation signal, initially the working connector (22₁ to 22ₙ) of an instrument (11, 12) that is to be activated is connected to the active output (15) of the HF generator (10), and then the HF generator (10) is controlled so that it supplies the working current,
wherein
the safety device (20) being designed and connected to the working connectors (22₁ to 22ₙ) as well as to the neutral output (16) in such a way that every working connector (22₁ to 22ₙ), except the working connector (22₁ to 22ₙ) of the instrument (11, 12) that is to be activated, is connected to the neutral output (16), wherein at the working connectors (22₁ to 22ₙ) there are provided sampling elements (33₁ to 33ₙ) for detecting the magnitude of a current and/or voltage, wherein the control means (21) is designed to switch off the HF generator (10) whenever the current and/or the voltage at the working connectors (22₁ to 22ₙ) that are connected to the neutral output exceeds a predetermined threshold.

2. The HF-surgery appliance according to Claim 1,
**characterized in that**
change-over switches (30₁ to 30ₙ) are provided for the purpose of switching a working connector (22₁ to 22ₙ) from the neutral output (16) to the active output (15) of the HF generator (10) and conducting a working current from the HF generator (10) to an instrument (11).

## Patentansprüche

1. HF-Chirurgieanordnung, welche aufweist:
- mindestens einen HF-Generator (10) mit einem aktiven Ausgang (15) und einem neutralen Ausgang (16) zur Erzeugung eines Arbeitsstromes,
- mindestens zwei Arbeits-Verbinder (22₁ bis 22ₙ) zum Anschluss mindestens eines Instruments (11, 12), durch die der Arbeitsstrom an biologisches Gewebe (1) geleitet werden kann,
- eine Sicherheitseinrichtung (20) zur Verringerung der durch Fehlerströme, die durch ein inaktives Instrument (12) fließen können, geschaffenen Gefahr, wobei die Sicherheitseinrichtung (20) eine Steuereinrichtung (21) aufweist, die derart gestaltet ist, dass in Reaktion auf ein Aktivierungssignal anfangs der Arbeits-Verbinder (22₁ bis 22ₙ) eines Instruments (11, 12), welches zu aktivieren ist, mit dem aktiven Ausgang (15) des HF-Generators (10) verbunden wird und dann der HF-Generator (10) so gesteuert wird, dass er den Arbeitsstrom liefert,
wobei
die Sicherheitseinrichtung (20) so gestaltet und mit den Arbeits-Verbindern (22₁ bis 22ₙ) sowie dem neutralen Ausgang (16) verbunden ist, dass jeder Arbeits-Verbinder (22₁ bis 22ₙ), außer dem Arbeits-Verbinder (22₁ bis 22ₙ) des zu aktivierenden Instruments (11, 12), mit dem neutralen Ausgang (16) verbunden ist, wobei bei den Arbeits-Verbindern (22₁ bis 22ₙ) Sampling-Elemente (33₁ bis 33ₙ) zur Erfassung der Größe eines Stroms und/oder einer Spannung vorgesehen sind, wobei die Steuereinrichtung (21) so gestaltet ist, dass sie den HF-Generator (10) immer ausschaltet, wenn der Strom und/oder die Spannung an den Arbeits-Verbindern (22₁ bis 22ₙ), die mit dem neutralen Ausgang verbunden sind, eine vorbestimmte Schwelle übersteigt.

2. HF-Chirurgieanordnung nach Anspruch 1,
**dadurch gekennzeichnet, dass**
Umschalter (30₁ bis 30ₙ) zum Zwecke des Schaltens eines Arbeits-Verbinders (22₁ bis 22ₙ) vom neutralen Ausgang (16) an den aktiven Ausgang (15) des HF-Generators (10) und zum Leiten eines Arbeitsstromes vom HF-Generator (10) an das Instrument (11) vorgesehen sind.

## Revendications

1. Appareil de chirurgie à haute fréquence (HF) comprenant :
- au moins un générateur HF (10) avec une sortie active (15) et une sortie neutre (16) pour générer un courant de travail,
- au moins deux connecteurs de travail (22₁ à 22ₙ) pour la connexion d'au moins un instrument (11, 12) via lequel le courant de travail peut être conduit à un tissu biologique (1),
- un dispositif de sécurité (20) pour réduire le danger présenté par des courants d'erreurs qui peuvent s'écouler à travers un instrument inactif (12), le dispositif de sécurité (20) comprenant un moyen de commande (21) conçu de telle façon que, en réponse à un signal d'activation, initialement le connecteur de travail (22₁ à 22ₙ) d'un instrument (11,12) qui doit être activé est connecté à la sortie active (15) du générateur HF (10), et ensuite le générateur HF (10) est commandé de telle façon qu'il fournit le courant de travail,
dans lequel
le dispositif de sécurité (20) est conçu et connecté au collecteur de travail (22₁ à 22ₙ), ainsi qu'à la sortie neutre (16) d'une telle manière que chaque connecteur de travail (22₁ à 22ₙ), excepté le connecteur de travail (22₁ à 22ₙ) de l'instrument (11, 12) qui doit être activé, est connectée à la sortie neutre (16), dans lequel, au niveau des connecteurs de travail (22₁ à 22ₙ) il est prévu des éléments d'échantillonnage (33₁ à 33ₙ) pour détecter l'amplitude d'un courant et/ou d'un voltage, dans lequel le moyen de commande (21) est conçu pour couper le générateur HF (10) chaque fois que le courant et/ou le voltage au niveau des connecteurs de travail (22₁ à 22ₙ) qui sont connectés à la sortie neutre excède un seuil prédéterminé.

2. Appareil de chirurgie HF selon la revendication 1,
**caractérisé en ce que**
des commutateurs inverseurs (30₁ à 30ₙ) sont prévus dans le but de commuter un connecteur de travail (22₁ à 22ₙ) depuis la sortie neutre (16) vers la sortie active (15) du générateur HF (10) et de conduire un courant de travail depuis le générateur HF (10) à un instrument (11).
